# EUROPEAN PATENT SPECIFICATION

(11) **EP 0 442 896 B1**
(45) Date of publication and mention of the grant of the patent: **15.05.1996**
(21) Application number: 89910904.5
(22) Date of filing: 28.09.1989
(51) Int. Cl.: B32B 27/04, B32B 27/12, E04D 11/02

(54) **LAMINATE AND PROCESS FOR PRODUCING SAME**
SCHICHTSTOFF UND VERFAHREN ZU SEINER HERSTELLUNG
STRATIFIE ET SON PROCEDE DE FABRICATION

(30) Priority: 03.10.1988 GB 8823144
(43) Date of publication of application: 28.08.1991
(73) Proprietor: ELLIOTT, Thomas George, Worcestershire WR9 0DW (GB); DUNNE, Desmond Charles, Ware, Hertfordshire SG12 0EA (GB); COLLINS, Michael Anthony, Del Terri, Gerona (ES)
(72) Inventor: Elliott, Thomas George, Droitwich, Worcestershire WR9 0DW (GB)
(74) Representative: Prentice, Raymond Roy
(86) International application number: GB8901154
(87) International publication number: WO9003887

(56) References cited:
- AU-A- 51 047
- CA-A- 916 543
- FR-A- 2 011 433
- FR-A- 2 355 964

## Description

This invention relates to a curable plastic sheet material particularly, but not exclusively, for use as a roofing material. A problem associated with flat roofed buildings is providing a satisfactory weatherproof and durable roofing.

Due to its strength, glass fibre has proved a popular roofing material. This is often used as follows:
a catalysed resin is first spread over the roof with a roller, glass fibre matting is then unrolled in strips onto the tacky resin, and the remainder of the catalysed resin is thereafter spread over and worked into the mat. Further details are available from Glassguard Products Limited of Sandycroft, Deeside.

The final roofing, however, often leaks and is inconsistent owing to, for example, a lack of skill of the roofer, or poor weather conditions when laying the roof.

It is an object of the present invention to provide a material, particularly a roofing material, which can be easily laid.

According to the first aspect of the present invention, there is provided a curable plastic sheet material characterised in that at least one layer of fibrous reinforcing material is laminated with a support material, both materials impregnated with a curable polymer composition including a radiation sensitive curing catalyst, the interstices of the support material being sufficiently small such that on curing, the sheet material will be substantially water impermeable.

Support refers to a structural support given to the polymer composition; and preferably the support material is on the surface of the reinforcing material.

Preferably also, the support material is a fibrous tissue, and more preferably the fibrous support material is fibre glass tissue.

Preferably also, the impregnated support material is between 20 and 40g/m², and more preferably the impregnated support material is about 30g/m². Preferably also, the curable polymer composition includes a polyester resin.

Preferably also, the radiation sensitive catalyst for curing is a catalyst responsive to UV radiation.

Preferably also the fibrous reinforcing and support materials are sandwiched between two protective sheets.

According to a second aspect of the invention, there is provided a process for the production of a curable plastic sheet material characterised in that at least one layer of fibrous reinforcing material is laminated on one surface of a support material, both of which materials are impregnated respectively with a first and second quantity of a curable polymer composition including a radiation sensitive curing catalyst, the interstices of the support material being sufficiently small such that on curing of said second quantity of polymer composition, the laminate sheet material will be substantially water impermeable.

Preferably a first and second outer protective sheet is further used, and the first and second quantities of the curable polymer composition respectively are applied to these, and more preferably, the curable polymer composition is tacky when applied to the respective outer protective sheets.

Preferably also the laminate is passed between a first and second set of rollers to exert a pressure and help impregnate the fibrous reinforcing and support material.

Preferably also, the support material is first sandwiched between the first and second quantities of curable polymer composition and then passed between the rollers.

It will, of course, be appreciated that the curable polymer composition, the support material, the fibrous reinforcing material and the outer protection sheets as defined in the first aspect of the invention are preferably used in the second aspect of the invention.

According to a third aspect of the invention there is provided the use of a curable plastic sheet material as defined in the first aspect of the invention or produced in accordance with the second aspect of the invention for construction and particularly for roofing.

Preferably radiation lamps (preferably UV lamps) are used to initially set the curable polymer composition so that it can withstand the rain.

A curable plastic sheet material embodying the invention, and a method of making the curable plastic sheet material, are hereinafter described, by way of example, with reference to the accompanying drawings.
Figure 1 is a cross-section view of part of a curable plastic sheet material in accordance with the invention;
Figure 2 is a schematic side elevational view of apparatus for carrying out a process for making the curable plastic sheet material of Figure 1.

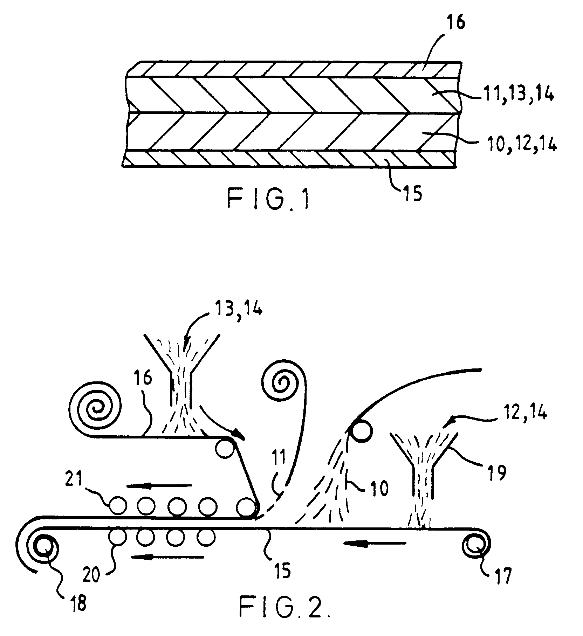

Referring to the drawings, Figure 1 shows a curable plastic sheet material comprising a reinforcing layer in the form of chopped "E" glass fibre 10, and a support layer in the form of glass fibre tissue 11 on the upper (as viewed) surface thereof, impregnated respectively with a first and second quantity of a UV curable polymer composition 12 to 14 and all sandwiched between a first and second outer protective sheet of suitable material such as nylon or melinex (trade mark) 15 and 16.

Expressed by weight of the impregnated "E" glass fibre in the reinforcing layer 10, the polymer composition 12, 14 includes 25% w/w isophthalic fire retardant polyester resin with an amount of benzoin butyl ether UV responsive catalyst, and 50% w/w inert fillers 14, such as colour pigments (optional), thickeners and fire retardants. The reinforcing "E" glass fibres 10 at 25% w/w, are 25mm long, and the impregnated layer 11, 13, 14 of glass fibre tissue is 30g/m².

Apparatus and process for making the curable plastic sheet material, is shown with reference to Figure 2. In the process the first nylon sheet 15 moves in the direction of the arrows (as shown) between rollers such as a first and second cardboard tube 17 and 18. The first quantity of UV curable polyester resin 12 is mixed and extruded with the inert fillers 14 from container 19 and applied to the upper surface of the first nylon sheet 15 to provide a tacky polymer composition.

The reinforcing "E" glass fibres 10 are then sprayed onto the tacky polymer composition 12, 14. The first nylon sheet 15 with reinforcing material 10 and polymer composition 12, 14 applied thereto is then passed to a first and second set of rollers 20 and 21.

Above the first and second set of rollers 20 and 21, the second nylon sheet 16 unwinds and has the second quantity of UV curable polyester resin 13 and the inert fillers 14 extruded onto its uppermost surface. By passing the prepared second nylon sheet 16 with resin and inert fillers 13, 14 applied thereto round two guide rollers, it is doubled back on itself, so inverting the surface carrying the tacky resin inert filler composition 13, 14. The glass fibre surface tissue 11, which forms the upper surface of the sheet material, is then brought into contact with the tacky surface of the resin and inert fillers on the second nylon sheet 16 and passes between the two sets of rollers 20 and 21. Under the pressure of the rollers the reinforcing material 10 is laminated with the surface tissue 11 and the first and second quantities of polyester resin impregnate the respective fibre glass layers 10 and 11.

With this construction, the curable plastic sheet material has the reinforcing strength of the "E" glass fibres 10, but it is also weather proofed through the impregnation of the fibre-glass tissue 11, by the resin inert filler composition 13, 14.

The curable plastic sheet material is applied to a roof as follows:-
(1) The laminate is first laid in strips onto a clean, sound, dry roof;
(2) With the fibre-glass surface tissue 11 uppermost, the laminate strips are lapped 2-3 inches (5.08-7.62 cm), and the intervening sheets of nylon pulled clear. The exposed, lapped resin is then treated with resin and the laps forced together with a metal roller to ensure good jointing;
(3) Finally the second (top) nylon sheet is stripped off and a thin coating of waterproofing compound applied to protect the sheet material until it has cured (in at least 24 hours). Preferably, however, the UV lamps are used to cure the polyester resin sufficiently to withstand the rain. The waterproof coating will, therefore, not be required, and the resin will still cure further under the natural sunlight.
(4) The sheet material is fixed by nails round the edges of the roof.

The invention has a number of advantages over hand applied roofings such as described hereinbefore.
(1) It is quicker to apply and since it requires much less application skill, the final roofing is better;
(2) It is not so dependent on the weather and can be laid in most conditions;
(3) Fewer hand tools are required;
(4) It can be walked on at any time;
(5) It can withstand most weather conditions within a few minutes.

The surface glass fibre tissue acts as a support matrix for the second quantity of polyester resin, and so any support material can be used which has sufficiently small interstices so that on impregnation with a curable polymer resin, a substantially water impermeable layer is formed.

Although polyester resin is most preferred, other suitable resins are, for example, epoxies and polyurethanes, or pvc. Furthermore the resin could be curable in a number of ways and the preferred UV curable method provides a sufficient shelf life of the sheet material. The first and second quantity can be different. It should be noted for example that the ratio of resin to reinforcing fibre-glass could be from about 4:1 to 1:4.

The curable plastic sheet material can also be used for purposes other than roofing, such as for lining reservoirs or for the construction of buildings, DIY vehicle repairs, casualty limb support via vacuum moulding.

The curable plastic sheet material may contain translucent colouring.

## Claims

1. A curable plastic sheet material characterised in that at least one layer (10) of fibrous reinforcing material is laminated with a support material (11), both materials (10, 11) being impregnated with a curable polymer composition (12, 14) including a radiation sensitive curing catalyst, the interstices of the support material (11) being sufficiently small such that on curing, the sheet material will be substantially water impermeable.

2. A curable plastic sheet material according to Claim 1, characterised in that the support material (11) is a fibrous tissue.

3. A curable plastic sheet material according to Claim 2, characterised in that the fibrous support material (11) comprises a glass fibre tissue.

4. A curable plastic sheet material according to any preceding Claim, characterised in that the impregnated support material (11) is between 20 and 40g/m².

5. A curable plastic sheet material according to Claim 4, characterised in that the impregnated support material (11) is about 30g/m².

6. A curable plastic sheet material according to any preceding Claim, characterised in that the curable polymer composition comprises a polyester resin.

7. A curable plastic sheet material according to any preceding Claim, characterised in that the catalyst is responsive to UV radiation.

8. A curable plastic sheet material according to any preceding Claim, characterised in that the impregnated reinforcing and support materials (10, 11) are sandwiched between two protective sheets (15, 16).

9. A process for the production of a curable plastic sheet material, characterised in that at least one layer of fibrous reinforcing material (10), is laminated on one surface of a support material (11), both of which materials (10, 11) are impregnated respectively with a first and second quantity of a curable polymer composition (12, 14; 13, 14), including a radiation sensitive catalyst, the interstices of the support material (11) being sufficiently small such that on curing of said second quantity of polymer composition, the sheet material will be substantially water impermeable.

10. A process according to Claim 9, characterised in that a first and second outer protective sheet (15, 16) is further used, and the first and second quantities of the curable polymer composition (12, 14; 13, 14) respectively are applied to these.

11. A process according to Claim 10, characterised in that the curable polymer composition (12, 14; 13, 14) is tacky when applied to the respective outer protective sheets (15, 16).

12. A process according to any of Claims 9 to 11, characterised in that the curable plastic sheet material is passed between a first and second set of rollers (20, 21) to exert a pressure and help impregnate the fibrous reinforcing and support material (10, 11).

13. A process according to Claim 12 when dependent on Claim 11, characterised in that the support material (11) is first sandwiched between the first and second quantities of curable polymer composition and then passed between the rollers (20, 21).

14. A process of covering a roof, characterised in that sheets of a curable plastic material as claimed in Claims 1 to 8 or produced in accordance with Claims 9 to 13 are laid upon the roof and the curable plastic material is cured in situ by exposure to radiation.

## Patentansprüche

1. Aushärtbares Kunststoff-Folienmaterial, dadurch gekennzeichnet, daß zumindest eine Lage (10) aus fasrigem Verstärkungsmaterial mit einem Trägermaterial (11) laminiert wird, wobei beide Materialien (10, 11) mit einer aushärtbaren Polymer-Zusammensetzung (12, 14) imprägniert sind, die einen strahlungsempfindlichen Aushärtkatalysator unifaßt, wobei die Zwischenräume des Trägermaterials (11) genügend klein sind, so daß aufgrund des Aushärtens das Folienmaterial im wesentlichen wasserundurchlässig ist.

2. Aushärtbares Kunststoff-Folienmaterial nach Anspruch 1, dadurch gekennzeichnet, daß das Trägermaterial (11) ein Fasergewebe ist.

3. Aushärtbares Kunststoff-Folienmaterial nach Anspruch 2, dadurch gekennzeichnet, daß das fasrige Trägermaterial (11) ein Glasfasergewebe aufweist.

4. Aushärtbares Kunststoff-Folienmaterial nach einem vorgenannten Anspruch, dadurch gekennzeichnet, daß das imprägnierte Trägermaterial (11) zwischen 20 und 40 g/m² liegt.

5. Aushärtbares Kunststoff-Folienmaterial nach Anspruch 4, dadurch gekennzeichnet, daß das imprägnierte Trägermaterial (11) bei etwa 30 g/m² liegt.

6. Aushärtbares Kunststoff-Folienmaterial nach einem vorgenannten Anspruch, dadurch gekennzeichnet, daß die aushärtbare Polymer-Zusammensetzung ein Polyester-Kunstharz umfaßt.

7. Aushärtbares Kunststoff-Folienmaterial nach einem vorgenannten Anspruch, dadurch gekennzeichnet, daß der Katalysator auf UV-Strahlung anspricht.

8. Aushärtbares Kunststoff-Folienmaterial nach einem vorgenannten Anspruch, dadurch gekennzeichnet, daß die imprägnierten Verstärkungs- und Trägermaterialien (10, 11) schichtweise zwischen zwei Schutzlagen (15, 16) angeordnet sind.

9. Verfahren zur Herstellung eines aushärtbaren Kunststoff-Folienmaterials, dadurch gekennzeichnet, daß zumindest eine Lage eines fasrigen Verstärkungsmaterials (10) auf eine Oberfläche des Trägermaterials (11) laminiert wird, daß beide diese Materialien (10, 11) jeweils mit einer ersten und zweiten Menge einer aushärtbaren Polymer-Zusammensetzung (12, 14; 13, 14) imprägniert werden, die einen strahlungsempflindlichen Katalysator umfaßt, wobei die Zwischenräume des Trägermaterials (11) genügend klein sind, so daß nach einer Aushärtung der zweiten Menge der Polymer-Zusammensetzung das Folienmaterial im wesentlichen wasserundurchlässig ist.

10. Verfahren nach Anspruch 9, dadurch gekennzeichnet, daß eine erste und eine zweite äußere Schutzlage (15, 16) weiterhin verwendet wird und die erste und zweite Menge der aushärtbaren Polymer-Zusammensetzung (12, 14; 13, 14) jeweils auf diese aufgebracht werden.

11. Verfahren nach Anspruch 10, dadurch gekennzeichnet, daß die aushärtbare Polymer-Zusammensetzung (12, 14; 13, 14) klebrig ist, wenn sie auf die jeweiligen äußeren Schutzlagen (15, 16) aufgebracht wird.

12. Verfahren nach einem der Ansprüche 9 bis 11, dadurch gekennzeichnet, daß das aushärtbare Kunststoff-Folienmaterial zwischen einem ersten und zweiten Satz von Walzen (20, 21) hindurchgeführt wird, um einen Druck auszuüben und die Imprägnierung des fasrigen Verstärkungs- und Trägermaterials (10, 11) zu unterstützen.

13. Verfahren nach Anspruch 12, unter Rückbeziehung auf Anspruch 11, dadurch gekennzeichnet, daß das Trägermaterial (11) zuerst schichtweise zwischen den ersten und zweiten Mengen der aushärtbaren Polymer-Zusammensetzung angeordnet und anschließend zwischen den Walzen (20, 21) hindurchgeführt wird.

14. Verfahren zur Abdeckung eines Daches, dadurch gekennzeichnet, daß Lagen aus aushärtbarem Kunststoffmaterial wie in den Ansprüchen 1 bis 8 beansprucht oder entsprechend den Ansprüchen 9 bis 13 hergestellt, auf das Dach aufgelegt und das aushärtbare Kunststoffmaterial in situ ausgehärtet wird, indem es einer Strahlung ausgesetzt wird.

## Revendications

1. Matériau en feuille de matière plastique durcissable, caractérisé en ce qu'au moins une couche (10) de matériau fibreux de renforcement est laminée avec un matériau de support (11), les deux matériaux (10, 11) étant imprégnés d'une composition polymère durcissable (12, 14) comprenant un catalyseur de durcissement sensible aux rayonnements, les interstices du matériau de support (11) étant suffisamment petits pour que, lors du durcissement, le matériau en feuille devienne sensiblement imperméable à l'eau.

2. Matériau en feuille de matière plastique durcissable selon la revendication 1, caractérisé en ce que le matériau de support (11) est un tissu fibreux.

3. Matériau en feuille de matière plastique durcissable selon la revendication 2, caractérisé en ce que le matériau de support fibreux (11) comprend un tissu de fibres de verre.

4. Matériau en feuille de matière plastique durcissable selon l'une quelconque des revendications précédentes, caractérisé en ce que le matériau de support imprégné (11) présente un poids compris entre 20 et 40 g/m².

5. Matériau en feuille de matière plastique durcissable selon la revendication 4, caractérisé en ce que le matériau de support imprégné (11) présente un poids d'environ 30 g/m².

6. Matériau en feuille de matière plastique durcissable selon l'une quelconque des revendications précédentes, caractérisé en ce que la composition polymère durcissable comprend une résine polyester.

7. Matériau en feuille de matière plastique durcissable selon l'une quelconque des revendications précédentes, caractérisé en ce que le catalyseur est sensible aux rayonnements ultraviolets.

8. Matériau en feuille de matière plastique durcissable selon l'une quelconque des revendications précédentes, caractérisé en ce que les matériaux imprégnés de renforcement et de support (10, 11) sont pris en sandwich entre deux feuilles de protection (15, 16).

9. Procédé de fabrication d'un matériau en feuille de matière plastique durcissable, caractérisé en ce qu'au moins une couche de matériau de renforcement fibreux (10) est laminée sur une première surface d'un matériau de support (11), ces deux matériaux (10, 11) sont imprégnés respectivement d'une première et d'une seconde quantités d'une composition polymère durcissable (12, 14 ; 13, 14), comprenant un catalyseur sensible aux rayonnements, les interstices du matériau de support (11) étant suffisamment petits pour que, lors du durcissement de ladite seconde quantité de composition polymère, le matériau en feuille devienne sensiblement imperméable à l'eau.

10. Procédé selon la revendication 9, caractérisé en ce que l'on utilise en outre une première et une seconde feuilles extérieures de protection (15, 16), et en ce que les première et seconde quantités de composition polymère durcissable (12, 14 ; 13, 14) sont respectivement appliquées sur celles-ci.

11. Procédé selon la revendication 10, caractérisé en ce que la composition polymère durcissable (12, 14 ; 13, 14) est pégueuse lorsqu'elle est appliquée sur les feuilles extérieures de protection respectives (15, 16).

12. Procédé selon l'une quelconque des revendications 9 à 11, caractérisé en ce que le matériau en feuille de matière plastique durcissable est passé entre un premier et un second jeux de rouleaux (20, 21) afin d'exercer une pression et d'aider à imprégner le matériau fibreux de renforcement et de support (10, 11).

13. Procédé selon la revendication 12 lorsqu'elle dépend de la revendication 11, caractérisé en ce que le matériau de support (11) est tout d'abord pris en sandwich entre les première et seconde quantités de composition polymère durcissable et est ensuite passé entre les rouleaux (20, 21).

14. Procédé de réalisation de la couverture d'un toit, caractérisé en ce que des feuilles d'un matériau de matière plastique durcissable tel que revendiqué dans les revendications 1 à 8 ou réalisé selon les revendications 9 à 13 sont déposées sur le toit, et le matériau de matière plastique durcissable est réticulé in situ par exposition aux rayonnements.
